# DEMANDE DE BREVET EUROPEEN

(11) **EP 1 405 626 A1**
(43) Date de publication de la demande: **07.04.2004**
(21) Numéro de dépôt: 03292287.4
(22) Date de dépôt: 17.09.2003
(51) Int. Cl.: A61K 7/06

(54) **Utilisation de latex cationiques non-filmogènes pour l'augmentation de volume de la chevelure**

(30) Priorité: 04.10.2002 FR 0212361
(71) Demandeur: L'OREAL, 75008 Paris (FR)
(72) Inventeur: Giroud, Franck, 92110 Clichy (FR)
(74) Mandataire: Bourdeau, Françoise

(57) **Abrégé**

L'invention concerne une composition de traitement capillaire comprenant, en dispersion dans un milieu liquide cosmétiquement acceptable, des particules de latex cationiques,
- formées d'un polymère synthétique, obtenu par polymérisation radicalaire, ayant une température de transition vitreuse (T_{g}) supérieure ou égale à 30 °C,
- ayant une taille moyenne de particules comprise dans l'intervalle allant de 10 nm à 200 nm, et
- ayant un potentiel superficiel zêta (ζ) supérieur à +20 mV
ainsi que l'utilisation d'une telle composition pour augmenter le volume de la chevelure.

## Description

La présente invention concerne l'utilisation de fines particules de latex cationiques non-filmogènes pour augmenter le volume de la chevelure, des compositions capillaires contenant de telles particules de latex cationiques et un procédé de traitement des cheveux utilisant ces compositions.

Il existe actuellement sur le marché un certain nombre de produits à base de polymères filmogènes qui permettent d'apporter du volume à la chevelure. Toutefois, le dépôt des tels polymères filmogènes à la surface des cheveux leur confère généralement un toucher peu naturel.
Une autre approche bien connue pour augmenter le volume de la coiffure est la déformation permanente des cheveux par un procédé utilisant successivement un agent réducteur et un agent oxydant. L'exposition des fibres kératiniques à de telles conditions physico-chimiques relativement agressives se traduit toutefois par une dégradation de la kératine et par une altération des propriétés mécaniques et cosmétiques des cheveux. Par ailleurs, ce type de procédé modifie également le niveau de frisure des cheveux ce qui n'est pas toujours un effet recherché par les personnes désireuses d'augmenter le volume de leur chevelure.
Il n'existe actuellement aucun traitement permettant d'accroître le volume de la chevelure sans modification de la forme des cheveux ou altération du toucher de ceux-ci.

La demanderesse a découvert qu'il était possible d'apporter du volume à la chevelure, sans dégradation des fibres kératiniques ni modification désagréable du toucher des cheveux, en traitant les cheveux avec une composition contenant un latex cationique non-filmogène tel que décrit ci-après. Les particules de latex, du fait de leur très petite taille et de leur charge cationique, se fixent sur les fibres kératiniques et y restent adsorbées sans toutefois former un film polymérique susceptible de modifier le toucher des cheveux traités.

La présente invention a par conséquent pour objet une composition de traitement capillaire comprenant, en dispersion dans un milieu liquide cosmétiquement acceptable, des particules de latex cationiques,
- formées d'un polymère synthétique, obtenu par polymérisation radicalaire, ayant une température de transition vitreuse (T_{g}) supérieure ou égale à 30 °C,
- ayant une taille moyenne de particules comprise dans l'intervalle allant de 10 nm à 200 nm, et
- ayant un potentiel superficiel zêta (ζ) supérieur à +20 mV
ainsi que l'utilisation d'une telle composition pour augmenter le volume de la chevelure.

On entend par latex dans la présente invention une suspension de particules en polymère synthétique, obtenue par polymérisation radicalaire en émulsion dans l'eau, généralement en présence d'agents tensioactifs. La polymérisation en émulsion est une technique de polymérisation bien connue et l'on pourra se référer pour de plus amples détails notamment à l'ouvrage "Latexes, Preparation, Characterisation and Applications", édité par E. S. Daniels aux éditions ACS Symposium Series (ISBN 0-8412-2305-X).
Les monomères formant les latex utilisés dans la présente invention peuvent être choisis parmi tous ceux connus dans la technique, susceptibles d'être polymérisés en émulsion aqueuse.
On peut citer à titre d'exemples de tels monomères les composés vinylaromatiques tels que le styrène, le méthylstyrène et le divinylbenzène, les monomères diéniques tels que le butadiène, le diméthylbutadiène, l'isoprène, le 1,3-hexadiène et le chloroprène, les acrylates et méthacrylates d'alkyle en C₁₋₁₀ ou d'aryle tels que les (méth)acrylates de méthyle, d'éthyle, de n-butyle, de 2-éthylhexyle, de t-butyle, isobornyle, de phényle et de benzyle, l'acétate de vinyle, l'acrylonitrile, le chlorure de vinyle et le chlorure de vinylidène, les alcènes tels que l'éthylène et le propylène, les monomères ou télomères fluorés tels que les (méth)acrylates de fluoroalkyle, et l'α-fluoroacrylate d'alkyle, l'acrylamide et ces dérivés alkylés tels que le N-méthylacrylamide et le N-isopropylacrylamide, le méthacrylamide, le (méth)acrylate de polyéthylèneglycol, le N-vinylacétamide, le N-méthyl-N-vinylacétamide, le N-vinylformamide, le N-méthyl-N-vinylformamide, les N-vinyllactames comportant un groupe alkyle cyclique en C₄₋₉, l'acide acrylique, l'acide méthacrylique, les (méth)acrylates d'hydroxyéthyle et d'hydroxypropyle.
Les monomères ci-dessus, doivent donner, après polymérisation, un polymère en dispersion dans l'eau dont la température de transition vitreuse est supérieure à 30 °C. Parmi ces polymères, on peut citer de façon non-exaustive, le polystyrène, le poly(acétate de vinyle), le poly(α-méthylstyrène), le poly(acrylamide), le poly(acrylonitrile), le poly(chlorure de vinyle), les copolymères à base de styrène et de (méth)acrylate d'alkyle en C₁₋₄, les copolymères à base de styrène et d'acrylamide, les copolymères à base de styrène et d'acrylonitrile, les copolymères à base de styrène et d'acétate de vinyle, les copolymères à base d'acrylamide et de (méth)acrylates d'alkyle en C₁₋₄, les copolymères à base d'acrylonitrile et de (méth)acrylate d'alkyle en C₁₋ ₄, les copolymères à base d'acrylonitrile et d'acrylamide, les terpolymères à base de styrène, d'acrylonitrile et d'acrylamide, le poly(méthacrylate de méthyle), le poly(méthacrylate d'éthyle), les copolymères styrène/butadiène, styrène/acide acrylique, styrène/vinylpyrrolidone et butadiène/acrylonitrile.

La taille moyenne des particules de latex de la présente invention doit être comprise entre 10 nm et 200 nm. Au-delà de cette limite supérieure, il est très difficile d'immobiliser les particules sur les fibres kératiniques et l'effet recherché, c'est-à-dire l'augmentation du volume de la chevelure, est insuffisant. La demanderesse a obtenu des résultats particulièrement satisfaisants avec des particules ayant une taille moyenne de particules comprise dans l'intervalle allant de 50 à 150 nm. On entend par "taille de particule" ici la dimension maximale qu'il est possible de mesurer entre deux points diamétralement opposés de la particule. Les techniques de mesure de la taille moyenne d'une suspension de particules sont l'observation directe par microscopie, notamment par microscopie à balayage électronique ou par microscopie à force atomique, mais on peut également utiliser des techniques indirectes bien connues telles que la diffusion dynamique de la lumière.
Les particules de latex de la présente invention ont de préférence une forme sphérique, mais il peut également s'agir de particules de forme aléatoire. Leur structure interne peut être de type noyau-enveloppe, en sandwich ou il peut s'agir de particules résultant de l'agglomération de sphères ("framboise"). La taille moyenne globale de tels agrégats de plusieurs sphères doit toutefois être comprise dans les limites indiquées ci-dessus. Pour plus de détails concernant les diverses structures de latex, on pourra se référer aux pages 235 - 238 de l'ouvrage précité "Latexes, Préparation, Characterisation and Applications".

Le caractère non-filmogène à température ambiante (environ 20 °C) des latex cationiques utilisés est une caractéristique essentielle des compositions de traitement capillaire de la présente invention. En effet, comme il est expliqué ci-dessus, les particules adsorbées ne doivent pas former un film polymérique à la surface de la fibre kératinique car cela entraînerait une modification indésirable du toucher des cheveux traités. Le caractère non-filmogène à température ambiante des polymères utilisables dans la présente invention résulte d'une température de transition vitreuse (Tg), mesurée par analyse calorimétrique différentielle (DSC, *Differential Scanning Calorimetry)* avec un gradient de température de 10 °C/minute, supérieure ou égale à 30 °C. Un tel polymère se trouvera, à température ambiante, dans un état vitreux qui ne permet pas la déformation plastique, indispensable à la formation d'un film.
Des latex cationiques utilisés de préférence dans la présente invention ont une température de transition vitreuse (T_{g}) supérieure à 40 °C, et en particulier supérieure à 50 °C.

Le caractère cationique des latex utilisés dans les compositions de la présente invention est exprimé par le potentiel zêta (ζ), a savoir par la différence de potentiel existant entre le plan de cisaillement (plan de coupure hydrodynamique) proche de la surface des particules et le sein du liquide de suspension. Pour rappel, ce plan de cisaillement se trouve au voisinage du plan extérieur d'Helmholtz, c'est-à-dire entre la couche rigide (couche de Stem) et la couche diffuse (couche de Gouy - Chapman) d'ions entourant les particules. Le potentiel zêta s'exprime en Volts, le millivolt étant l'unité la plus couramment utilisée en raison des faibles valeurs de différence de potentiel mesurées. Le potentiel zêta est déterminé classiquement par électrophorèse, c'est-à-dire par mesure de la vitesse de déplacement des particules dans un champ électrique. Cette vitesse est déterminée par diffusion de la lumière avec effet Doppler. De tels appareils de mesure du potentiel zêta (électrophorèse + laser à effet Doppler) sont commercialisés notamment par la société MALVERN INSTRUMENT sous la dénomination Zetasizer ou Zetamaster.
Pour plus de détails concernant la définition du potentiel zêta et la mesure de celui-ci, on pourra se référer à l'ouvrage intitulé "Phénomènes d'interfaces, Agents de surface" de J. Briant, publié aux éditions Technip (ISBN 2.7108.0578-2) respectivement aux pages 165 - 203 et aux pages 223 - 243.
La valeur du potentiel zêta dépend fortement des conditions de mesure et notamment de la composition du milieu de suspension (force ionique, pH). Toutes les valeurs de potentiel zêta indiquées dans la présente demande ont été mesurées dans les conditions standard suivantes :
- appareil de mesure : Zetamaster, société MALVERN,
- suspension de particules à 50 ppm de matière active (latex) dans de l'eau déminéralisée Milli-Q,
- pH = 7, ajusté par addition d'HCl ou de KOH,
- force ionique du milieu fixée par addition de 1,3 mM/l de KCl,
- température : 25 °C.

Le potentiel zêta des latex cationiques utilisés dans les compositions de la présente invention est de préférence supérieur à +40 mV, et en particulier supérieur à +60 mV.

Il existe différentes techniques pour introduire des charges cationiques dans des latex de polymères synthétiques parmi lesquelles on peut citer :
- l'utilisation d'amorceurs radicalaires cationiques tels que l'azobisbutyroamidinium et le dichlorhydrate de 2,2'-azo-bis(2-amidinopropane) ; cette technique qui aboutit à l'incorporation de charges cationiques à l'extrémité des chaînes du polymère ne permet généralement pas d'obtenir une densité de charges très élevée à la surface des particules et doit généralement être utilisée en combinaison avec d'autres méthodes ;
- l'utilisation d'agents tensioactifs cationiques tels que le bromure de dodécyltriméthylammonium ; cette technique permet l'obtention de densités de charges importantes qui sont toutefois simplement adsorbées à la surface des particules ;
- la copolymérisation, en combinaison avec les monomères non-ioniques énumérés ci-dessus, d'une certaine fraction de comonomères cationiques insolubles dans l'eau qui se trouvent ainsi incorporés dans chaîne macromoléculaire du polymère formant les particules ; on peut citer à titre d'exemples de tels comonomères cationiques, les méthacrylates de N,N-dialkylaminoéthyle et de N,N-dialkylaminopropyle éventuellement quaternisés par un groupe alkyle en C₁₋₆ ; la synthèse d'un tel latex cationique par copolymérisation d'un monomère non ionique (styrène) et d'un monomère cationique (méthacrylate de N,N-diéthylaminoéthyle) est décrite par exemple dans l'article de B. Alince *et al.* dans *Journal of Applied Polymer Science, 76,* 1677 - 1682 (2000), et enfin
- le greffage, c'est-à-dire la fixation covalente, d'un composé organique cationique à la surface des particules de latex déjà formées. Les composés organiques cationiques peuvent être des composés de faible masse ou des polymères. On peut citer à titre d'exemple de la fixation d'un polymère cationique par greffage, la fixation d'une polyéthylèneimine thiolée sur un latex dont la surface a préalablement été fonctionnalisée par des groupes thiol, selon le schéma réactionnel suivant :

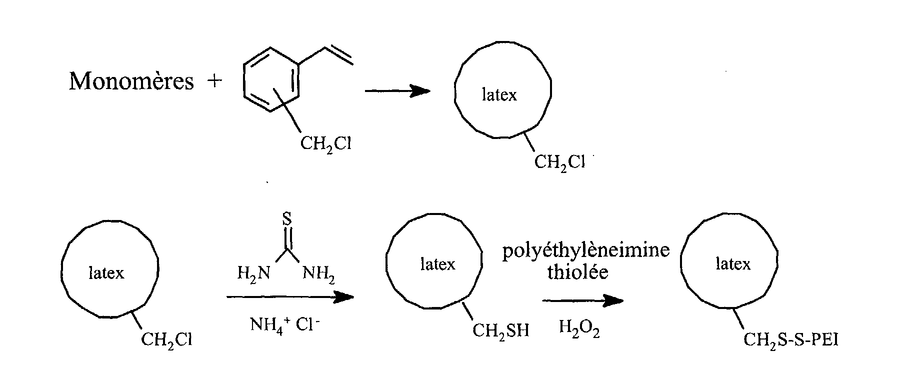

La concentration des particules de latex cationiques dans les compositions de la présente invention est de préférence comprise entre 0,001 % et 50 % en poids, en particulier entre 0,05 % et 5 % en poids et tout particulièrement entre 0,1 % et 2 % en poids, rapportée au poids total de la composition.
On entend par "milieu cosmétiquement acceptable", un milieu ne contenant pas d'ingrédients incompatibles, pour des raisons toxicologiques, avec une application sur le corps humain, et notamment sur la peau et les phanères.
Le milieu liquide cosmétiquement acceptable est de préférence un milieu aqueux contenant éventuellement une certaine fraction d'un ou de plusieurs solvants organiques tels que les alcanols inférieurs, en particulier l'éthanol et l'isopropanol, l'alcool benzylique, les polyols tels que le glycérol, les alkylèneglycols et polyalkylèneglycols tels que le propylèneglycol, le dipropylèneglycol et le butylèneglycol, l'acétone, la méthyléthylcétone, l'acétate de méthyle, l'acétate d'éthyle, le diméthoxyéthane ou le diéthoxyéthane.

Les compositions de traitement capillaires de la présente invention peuvent contenir en outre un ou plusieurs principes actifs et/ou adjuvants de formulation connus tels que des silicones, volatiles ou non, des huiles minérales, organiques ou végétales, des cires oxyéthylénées ou non, des paraffines, des alcanes en C₅₋₁₀, des acides gras, des amides gras, des esters gras, des alcools gras, des agents réducteurs, des agents oxydants, des séquestrants, des agents épaississants, des agents adoucissants, des agents anti-mousse, des agents hydratants, des agents émollients, des agents d'ajustement et de fixation du pH, des filtres solaires, des colorants directs ou des précurseurs de colorants d'oxydation, des pigments, des agents nacrants, des agents peptisants, des conservateurs, des agents tensioactifs, des polymères fixants, des agents plastifiants, des polymères conditionneurs, des protéines et des vitamines.
L'homme du métier, en choisissant ces adjuvants, veillera en particulier à ce qu'ils ne nuisent pas aux qualités intrinsèques des compositions de l'invention.

Les compositions selon la présente invention peuvent être conditionnées dans des dispositifs aérosol et contiennent alors un agent propulseur. On peut utiliser n'importe quel gaz comprimé ou liquéfié couramment employé pour la fabrication de compositions aérosol. On utilisera de préférence le gaz carbonique, l'azote comprimé, le diméthyléther, les hydrocarbures, les hydrocarbures halogénés, en particulier fluorés, et des mélanges de ces agents propulseurs.

Les compositions de la présente invention peuvent se présenter sous la forme d'une lotion, d'un spray, d'un spray aérosol, d'une mousse aérosol, d'un après-shampooing ou d'un shampooing.

L'invention a en outre pour objet un procédé de traitement capillaire comprenant
- l'application sur les cheveux, secs ou humides, d'une composition contenant, en dispersion dans un milieu liquide cosmétiquement acceptable, des particules de latex cationiques telles que définies ci-avant,
- éventuellement une étape de rinçage des cheveux, et
- le séchage des cheveux.
Le demanderesse a découvert que les latex cationiques utilisés dans les compositions de traitement de la présente invention avaient une excellente affinité pour les cheveux qui permettait de les utiliser non seulement en application directe sans rinçage mais également par exemple sous forme de shampooing ou d'après-shampooing en application rincée.
Le séchage des cheveux peut se faire à l'air libre à température ambiante mais également avec un apport de chaleur sous forme de rayonnement ou d'air chaud.

### Exemple 1

### Lotion coiffante

On prépare la lotion coiffante A selon l'invention et la lotion témoin B suivantes :

| ingrédient | composition A (selon l'invention) | composition B (témoin) |
|---|---|---|
| latex cationique* | 1,0 g de matière active | - |
| aminométhylpropanol | q.s.p. pH 7 | q.s.p. pH 7 |
| éthanol | 10g | 10g |
| parfum | q.s. | q.s. |
| eau | q.s.p. 100 g | q.s.p. 100 g |

| | | |
|---|---|---|
| * BASOPLAST® 265 D, BASF | | |

Le latex cationique (BASOPLAST 265 D, BASF) utilisé dans la composition A selon l'invention présente un potentiel zêta de 48 mV ± 1 mV, une température de transition vitreuse, mesurée par DSC, de 50 °C ± 1 °C et une taille moyenne de particules (moyenne en nombre, déterminée par diffusion dynamique de la lumière à l'aide d'un laser He-Ne et corrélateur Brookhaven BI9000) de 75 nm.

On applique 5 g de chacune des lotions coiffantes ci-dessus (compositions A et B) respectivement sur 10 chevelures. Les caractéristiques cosmétiques des coiffures sont évaluées par un groupe de 10 experts. L'ensemble des experts indiquent, de façon systématique, que l'application de la composition selon l'invention (composition A) contenant un latex cationique, apporte plus de volume à la chevelure que la composition témoin (composition B) et n'altère pas le toucher naturel des cheveux.

### Exemple 2

### Mousse de coiffage

On prépare les deux compositions C et D suivantes

| ingrédient | composition C (selon l'invention) | composition D (témoin) |
|---|---|---|
| Latex cationique de l'ex. 1 | 1 g m.a.* | - |
| Acétate d'amidon | 5 g m.a.* | 5 g m.a.* |
| Polysorbate 20 | 0,1 g m.a. * | 0,1 g m.a.* |
| Cocamidopropylbétaïne | 0,5 g m.a.* | 0,5 g m.a.* |
| Laureth-4 | 0,3 g m.a.* | 0,3 g m.a.* |
| Mélange Isobutane/butane/propane*** | 5 g m.a.* | 5 g m.a.* |
| Conservateur | q.s. | q.s. |
| Parfum | q.s. | q.s. |
| Eau | q.s.p.100 g | q.s.p.100 g |

| | | |
|---|---|---|
| *m.a. = matière active ** AERON 3.2 de la société SOLVADIS | | |

On applique 5 g de chacune des mousses de coiffage ci-dessus (compositions C et D ci-dessus) respectivement sur 10 chevelures. Les caractéristiques cosmétiques des coiffures sont évaluées par un groupe de 10 experts. L'ensemble des experts indiquent, de façon systématique, que l'application de la composition selon l'invention (composition C) contenant un latex cationique, apporte plus de volume à la chevelure que la composition témoin (composition D) sans altérer le toucher naturel des cheveux.

### Exemple 3

### Shampooing

On prépare les compositions de shampooing E et F ayant la composition suivante :

| ingrédient | Composition E (selon l'invention) | Composition F (témoin) |
|---|---|---|
| Latex cationique de l'exemple 1 | 1 g m.a.^{b)} | 1 g m.a.^{b)} |
| Sodium Laureth sulfate^{a)} | 20 g m.a.^{b)} | 20 g m.a.^{b)} |
| Cocobetaine^{a)} | 4 g m.a.^{b)} | 4 g m.a.^{b)} |
| Cocamide MIPA^{a)} | 2 g m.a.^{b)} | 2 g m.a.^{b)} |
| Sodium Cetearyl Sulfate^{a)} | 0,8 g m.a. ^{b)} | 0,8 g m.a.^{b)} |
| Carbomer^{a)} | 0,2 g m.a.^{b)} | 0,2 g m.a.^{b)} |
| Dimethicone^{a)} | 2 g m.a.^{b)} | 2 g m.a.^{b)} |
| Conservateur | q.s. | q.s. |
| Parfum | q.s. | q.s. |
| Eau | q.s.p.100 g | q.s.p.100 g |

| | | |
|---|---|---|
| ^{a)} dénomination CTFA Cosmetic Ingredient Handbook | | |
| ^{b)} m.a. = matière active | | |

On applique 5 g de chacune des compositions de shampooing E et F respectivement sur 10 chevelures de cheveux caucasiens naturels châtains de 20 cm de long et, après un temps de pose de 2 minutes, on les rince à l'eau et on les sèche au sèche-cheveux. Un groupe de 10 experts indiquent que, dans tous les cas, l'application de la composition selon l'invention (composition E) contenant un latex cationique apporte plus de volume à la chevelure que la composition témoin (composition F) sans altérer le toucher naturel des cheveux.
Ce dernier exemple montre que les compositions selon l'invention sont efficaces du point de vue de l'augmentation du volume de la chevelure même en application rincée.

## Revendications

1. Composition de traitement capillaire comprenant, en dispersion dans un milieu liquide cosmétiquement acceptable, des particules de latex cationiques,
• formées d'un polymère synthétique, obtenu par polymérisation radicalaire, ayant une température de transition vitreuse (T_{g}) supérieure ou égale à 30 °C,
• ayant une taille moyenne de particules comprise dans l'intervalle allant de 10 nm à 200 nm, et
• ayant un potentiel superficiel zêta (ζ) supérieur à +20 mV.

2. Composition selon la revendication 1, **caractérisée par le fait que** la taille moyenne des particules de latex est comprise entre 50 et 150 nm.

3. Composition selon la revendication 1 ou 2, **caractérisée par le fait que** la température de transition vitreuse (T_{g}) du polymère formant les particules de latex est supérieure à 40 °C, de préférence supérieure à 50 °C.

4. Composition selon l'une quelconque des revendications précédentes, **caractérisée par le fait que** le polymère synthétique formant les particules de latex est choisi parmi les homopolymères et copolymères à base de monomères vinylaromatiques, de monomères diéniques, d'acrylates et de méthacrylates d'alkyle en C₁₋₁₀ ou d'aryle, d'acétate de vinyle, d'acrylonitrile, de chlorure de vinyle, de chlorure de vinylidène, d'alcènes, de monomères ou télomères fluorés, d'acrylamide et ces dérivés alkylés, de méthacrylamide, de (méth)acrylate de polyéthylèneglycol, de N-vinylacétamide, de N-méthyl-N-vinylacétamide, de N-vinylformamide, de N-méthyl-N-vinylformamide, de N-vinyllactames comportant un groupe alkyle cyclique en C₄₋₉, d'acide acrylique, d'acide méthacrylique, de (méth)acrylate d'hydroxyéthyle et d'hydroxypropyle.

5. Composition selon l'une des revendications précédentes, **caractérisée par le fait que** le potentiel zêta (ζ) des particules de latex est supérieur à +40 mV, de préférence supérieur à +60 mV.

6. Composition selon l'une des revendications précédentes, **caractérisée par le fait que** la charge cationique des particules de latex est introduite
• par utilisation d'un amorceur cationique,
• par utilisation d'agents tensioactifs cationiques lors de la polymérisation en émulsion,
• par copolymérisation de comonomères cationiques, et/ou
• par greffage covalent d'un composé organique cationique à la surface des particules de latex.

7. Composition selon la revendication 6, **caractérisée par le fait que** l'on utilise, en tant qu'amorceur cationique, l'azobis-butyroamidinium.

8. Composition selon la revendication 6, **caractérisé par le fait que** l'on utilise, en tant qu'agent tensioactif, le bromure de dodécyltriméthylammonium.

9. Composition selon la revendication 6, **caractérisé par le fait que** l'on utilise, comme comonomères cationiques, les méthacrylates de N,N-dialkylaminoéthyle et de N,N-dialkylaminopropyle éventuellement quaternisés par un groupe alkyle en C₁₋₆.

10. Composition selon la revendication 6, **caractérisée par le fait que** le composé organique greffé à la surface des particules de latex est une polyéthylèneimine thiolée (PEI-SH).

11. Composition selon l'une quelconque des revendications précédentes, **caractérisée par le fait qu'**elle contient de 0,001 % à 50 % en poids, de préférence de 0,05 % à 5 % et en particulier de 0,1 % à 2 % en poids de particules de latex cationiques, rapporté au poids total de la composition.

12. Composition selon l'une quelconque des revendications précédentes, **caractérisée par le fait qu'**elle se présente sous la forme d'une lotion, d'un spray, d'un spray aérosol, d'une mousse aérosol, d'un après-shampooing ou d'un shampooing.

13. Procédé de traitement capillaire comprenant
• l'application sur les cheveux, secs ou humides, d'une composition selon l'une quelconque des revendications précédentes,
• éventuellement le rinçage des cheveux, et
• le séchage des cheveux.

14. Utilisation d'une composition selon l'une quelconque des revendications 1 à 12 pour augmenter le volume de la chevelure.
